# EUROPEAN PATENT SPECIFICATION

(11) **EP 0 945 437 B1**
(45) Date of publication and mention of the grant of the patent: **02.11.2006**
(21) Application number: 98105673.2
(22) Date of filing: 27.03.1998
(51) Int. Cl.: C07D 231/12, C07D 231/16, C07D 231/14, C07D 231/18, C07D 231/20, C07D 405/04, C07D 401/12, C07D 403/12, C07D 417/12, C07D 231/54, C07D 401/04, C07D 409/04, C07D 403/04, C07D 417/04, A61K 31/415, A61K 31/44, A61K 31/505, A61K 31/425

(54) **Pyrazolyl acrylic acid- and pyrazolyl oximino-acetic acid derivatives, their preparation and their use as fungicides**
Pyrazolylacrylsäure- und Pyrazolyloximinoessigsäure Derivate, deren Herstellung und deren Verwendung als Fungizide
Dérivés de pyrazolyl acide acrylique et parazolyl acide oximino-acetique, leur préparation et leur utilisation comme fongicides

(43) Date of publication of application: 29.09.1999
(73) Proprietor: SDS Biotech K.K., Minato-ku, Tokyo 105-0021 (JP)
(72) Inventor: Hirohara, Yoji, c/o Tsukuba Technology Center, Tsukuba-shi, Ibaraki, 300-2646 (JP); Sugano, Shigeyoshi, c/o Tsukuba Technology Center, Tsukuba-shi, Ibaraki, 300-2646 (JP); Nakashima, Hideki, c/o Tsukuba Technology Center, Tsukuba-shi, Ibaraki, 300-2646 (JP); Kimura, Takuo, c/o Tsukuba Technology Center, Tsukuba-shi, Ibaraki, 300-2646 (JP); Sakakibara, Takashi, c/o Tsukuba Technology Center, Tsukuba-shi, Ibaraki, 300-2646 (JP)
(74) Representative: Strehl Schübel-Hopf & Partner

(56) References cited:
- EP-A- 0 206 523
- EP-A- 0 433 899
- EP-A- 0 471 262
- EP-A- 0 532 126
- EP-A- 0 532 127
- EP-A- 0 579 071
- WO-A-94/29276
- CHEMICAL ABSTRACTS, vol. 120, no. 11, 14 March 1994 Columbus, Ohio, US; abstract no. 134461f, ODA M. ET AL.: "Preparation of 2-(5-pyrazolyl or 2-pyridyl)-2-(methoxyimino)acetic acid derivatives as agricultural and horticultural fungicides" page 041; column 1; XP002072537 & JP 05 201980 A (MITSUBISHI CHEMICAL INDUSTRY) 10 August 1993
- CHEMICAL ABSTRACTS, vol. 124, no. 8, 19 February 1996 Columbus, Ohio, US; abstract no. 87005f, ODA M. ET AL.: "Preparation of pyrazolylacetic acid derivatives as agrochemical fungicides" page 1313; column 1; XP002072538 & JP 07 224041 A (MITSUBISHI KAGAKU K.K.) 22 August 1995
- CHEMICAL ABSTRACTS, vol. 124, no. 21, 20 May 1996 Columbus, Ohio, US; abstract no. 289528q, OOSUMI T. ET AL.: "Preparation of 2-(5-pyrazolyloxy)acetic aceid derivatives as agrochemical fungicides" page 1344; column 1; XP002072539 & JP 07 330735 A (SUMITOMO CHEMICAL CO.) 19 December 1995

## Description

### BACKGROUND OF THE INVENTION

### 1. Field of the Invention

The present invention relates to novel pyrazole derivatives, preparation thereof, and fungicides in agriculture and horticulture and for industrial materials which contain the said derivatives as active ingredients.

### 2. Description of the Related Art

A variety of fungicides have so far been developed; however, they are not necessarily satisfactory for their effects, a development of the resistant strains thereof and the like.

European Patent Application Laid-open No. 206523 describes that certain pyrazole derivatives have fungicidal activity, but the compounds described in it are not satisfactory in view of the effect, residual effectiveness, phytotoxicity and the like, and accordingly, it has been desired to develop a fungicide in agriculture and horticulture which is further useful against damage by disease of plants.

EP 0206523 A1, EP 0532126 A1, EP 0433899 A1, EP 0471262 A1, EP 0532127 A1, EP 0579071 A1, JP 5-201980, JP 7-224041, JP 7-330735 and WO 94/29276 disclose heteroaromatic compounds with fungicidal activity.

### SUMMARY OF THE INVENTION

In view of such situations, the present inventors have been intensively studied to develop a compound which has excellent fungicidal activity, to find that substituted pyrazole derivatives represented by the following general formula (I) have excellent fungicidal activity and to complete the present invention.

That is, the present invention relates to novel substituted pyrazole derivatives, preparation thereof, and fungicides in agriculture and horticulture and for industrial materials which contain the derivatives as active ingredients shown below.
[1] A compound having following general formula (I): 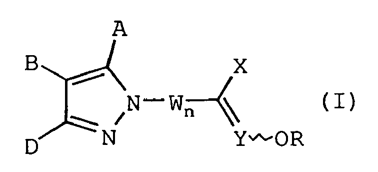 wherein
   X represents R¹OOC, R¹HNOC, R¹R¹NOC, CN or a 5- or 6-membered heteroaromatic group;
   Y represents CH or N;
   W represents C₁₋₃ alkylene, NR¹, or O;
   n is 1;
   R represents C₁₋₄ lower alkyl which may be optionally substituted by one or more same or different halogen atoms; and
   A, B and D represent same or different atom or group selected from hydrogen atom, halogen atoms, R¹, R¹O, R¹S, R¹SO, R¹SO₂, (R¹)₂N, R¹OOC, R¹OR², R¹ON=CH, CN, NO₂, C₂₋₄ lower alkenyl, C₂₋₄ lower alkynyl, C₃₋₇ cycloalkyl, Ph, PhCH₂, PhO, PhCH₂O, PhOR², PhS, PhCH₂S, PhSR², PhCH₂ON=CH, Naph, and Het,
   wherein R¹ represents C₁₋₄ lower alkyl which may be optionally substituted by one or more same or different halogen atoms, R² represents C₁₋₄ lower alkylene, Ph represents phenyl which may be optionally substituted by one to five group (s) selected from R¹, R¹O, R¹S, R¹SO, R¹SO₂, (R¹)₂N, R¹OOC, C₃₋₇ cycloalkyl, CN, NO₂, and halogen atoms, or by one Ph, PhCH₂, PhO, PhCH₂O, PhCH₂ON=C(R¹), PhCO, and Het-O; wherein R¹ represents the same as above; Het represents a 5- or 6-membered heteroaromatic group, and two of substituents R¹ and/or R¹O at the vicinal positions may form ring structure with the benzene ring; Naph represents naphthyl which may be optionally substituted by one or two group(s) selected from halogen atoms, R¹ and R¹O, and Het represents the same as above,
   provided that A, B and D do not represent hydrogen atom at the same time and more than two of A, B and D do not represent a group containing aromatic or heteroaromatic group at the same time.
[2] A compound as described in [1] above, wherein W represents a C₁₋₃ alkylene group.
[3] A compound as described in [1] or [2] above, wherein W represents a methylene group.
[4] A compound as described in [2] above, wherein X represents R¹OOC or R¹HNOC and Y represents CH₁ R¹ being defined as in claim 1.
[5] A fungicide in agriculture and horticulture and for industrial materials containing one of more kinds of the compounds as claimed in any one of [1] to [4] above as active ingredients.

### DETAILED DESCRIPTION OF THE PREFERRED EMBODIMENTS

### [Substituted pyrazole derivatives]

In general formula (I), W represents a C₁₋₃ alkylene, NR¹ or oxygen atom (O), and n is 1. Among them, the preferred is the case that n is 1 and W represents a C₁₋₃ alkylene group. More preferred is that n is 1 and W represents a methylene group.

In general formula (I), X represents R¹OOC, R¹HNOC, R¹R¹NOC, CN or a 5- or 6-membered heteroaromatic group.

The example of R¹OOC includes a methoxycarbonyl group, an ethoxycarbonyl group and the like, and preferably, a methoxycarbonyl group.

Examples of R¹HNOC and R¹R¹NOC include a methylaminocarbonyl group, an ethylaminocarbonyl group, a dimethylaminocarbonyl group, a diethylaminocarbonyl group, an ethylmethylcarbonyl group, and preferably, a methylaminocarbonyl group.

Examples of the 5- or 6-membered heteroaromatic group include 2-pyridyl, 3-pyridyl, 4-pyridyl, 2-furyl, 3-furyl, 2-thienyl and 3-thienyl.

In general formula (I), Y represents CH or a nitrogen atom.

When Y represents CH, it is preferable that n is 1 and W represents a C₁₋₃ alkylene group, and X represents desirably R¹OOC or R¹HNOC, and especially, a methoxycarbonyl group, a methylaminocarbonyl group are preferable.

In general formula (I), R represents a C₁₋₄ lower alkyl group which may be optionally substituted by one or more same or different halogen atoms, and preferably, a methyl, fluoromethyl or difluoromethyl group.

In general formula (I), the groups represented by A, B and D may be divided into (1) substituents not having a phenyl group, (2) substituents having phenyl group(s), and (3) heteroaromatic groups, and A, B and D do not represent hydrogen at the same time and more than two of A, B and D do not represent a group containing aromatic or heteroaromatic groups at the same time.

The preferable combinations of A, B and D are such that one of them represents a substituent having phenyl group (s) (2) or a heteroaromatic group (3), and the other two represent independently a substituent not having a phenyl group (1).

The substituent not having a phenyl group (1) represented by A, B and D means a hydrogen atom or halogen atoms, R¹, R¹O, R¹S, R¹SO, R¹SO₂, (R¹)₂N, R¹OOC, R¹OR², R¹ON=CH, CN, NO₂, C₂₋₄ lower alkenyl, C₂₋₄ lower alkynyl or C₃₋₇ cycloalkyl group wherein R¹ represents a C₁₋₄ lower alkyl group which may be optionally substituted by one or more same or different halogen atoms.

The examples of the C₁₋₄ lower alkyl group represented by R¹ include methyl, ethyl, propyl and butyl, and the isomeric groups thereof. Those of the C₃₋₇ cycloalkyl group include cyclopropyl, cyclobutyl, cyclopentyl, cyclohexyl and cycloheptyl. Those of R¹O include methoxy, ethoxy, propoxy and butoxy, and the isomeric groups thereof. Those of R¹S include methylthio, ethylthio, propylthio and butylthio, and the isomeric groups thereof. Those of R¹SO include methylsulfinyl, ethylsulfinyl, propylsulfinyl and butylsulfinyl, and the isomeric groups thereof. Those of R¹SO₂ include methylsulfonyl, ethylsulfonyl, propylsulfonyl and butylsulfonyl, and the isomeric groups thereof. Those of (R¹)₂N include dimethylamino, diethylamino, dipropylamino, dibutylamino and ethylmethylamino, and the isomeric groups thereof. Those of R¹OOC include methoxycarbonyl, ethoxycarbonyl, propoxycarbonyl and butoxycarbonyl, and the isomeric groups thereof. Those of the C₂₋₄ lower alkenyl group include vinyl, propenyl and butenyl, and the isomeric groups thereof. Those of the C₂₋₄ lower alkynyl group include ethynyl, propynyl and butynyl, and the isomeric groups thereof. Those of R¹ON=CH include a methoxyiminomethyl group. Those of R¹OR² include methoxymethyl, ethoxymethyl, propoxymethyl and butoxymethyl, and the isomeric groups thereof. The example of the halogen atoms includes fluorine, chlorine, bromine and iodine atoms. In the above, the C₁₋₄ lower alkyl group represented by R¹ may be substituted by one or more same or different halogen atoms.

The substituents having phenyl group (s) represented by A, B and D mean Ph, PhCH₂, PhO, PhCH₂O, PhOR², PhS, PhCH₂S, PhSR², PhCH₂ON=CH or Naph, wherein Ph represents a phenyl group which may be optionally substituted by one to five group(s) selected from R¹, R¹O, R¹S, R¹SO, R¹SO₂, (R¹)₂N, R¹OOC, CN, NO₂, a C₃₋₇ cycloalkyl group and halogen atoms, or by one group selected from Ph, PhCH₂, PhO, PhCH₂O, PhCH₂ON=C(R¹), PhCO and Het-O; wherein Het represents a 5- or 6-membered heteroaromatic group, and two of substituents R¹ and/or R¹O at the vicinal positions may form ring structure with the benzene ring; and Naph represents a naphthyl group which may be optionally substituted by one or two group (s) selected from halogen atoms, R¹ and R¹O.

The examples of the two of substituents R¹ and/or R¹O at the vicinal positions which may form ring structure with the benzene ring include 1,3-benzodioxol-4-yl, 2,2-dimethyl-1,3-benzodioxol-4-yl and 2,2-difluoro-1,3-benzodioxol-4-yl.

Among them, the preferred are phenyl, methylphenyl, chlorophenyl, dichlorophenyl, 3-[1-(chlorobenzyloxyimino)ethyl]phenyl, 3-(benzyloxy)phenyl, 3-(methylbenzyloxy)phenyl, 3-(chlorobenzyloxy)phenyl, 3-(cyanobenzyloxy)phenyl, 3-(dimethylbenzyloxy)phenyl, 3-(dichlorobenzloxy)phenyl, 3-(pyridylmethoxy)phenyl, 3-(benzoyloxy)phenyl, 3-(chlorobenzoyloxy)phenyl, 3-(6-chloropyrimidin-4-yloxy)phenyl, 3-(6-methoxypyrimidin-4-yloxy)phenyl, 3-[6-(2-methylphenoxy)-pyrimidin-4-yloxy]phenyl, [6-(2-cyanophenoxy)pyrimidin-4-yloxy]phenyl, 3-(6-chloro-5-nitropyrimidin-4-yloxy)phenyl, 3-(2-benzothiazolyloxy)phenyl, benzyl, phenoxymethyl, methylphenoxymethyl, phenylthiomethyl, methylphenylthiomethyl, 1-phenoxyethyl, 1-(methylphenoxy)ethyl, 1-phenylthioethyl, 1-(methylphenylthio)ethyl, 1,3-benzodioxol-4-yl, 2,2-dimethyl-1,3-benzodioxol-4-yl, 2,2-difluoro-1,3-benzodioxol-4-yl, phenoxy, 2-methylphenoxy, 3-methylphenoxy, 4-methylphenoxy, 2-chlorophenoxy, 3-chlorophenoxy, 4-chlorophenoxy, 2-trifluoromethylphenoxy, 2,5-dimethylphenoxy, 2,5-dichlorophenoxy, 2-chloro-5-trifluoromethylphenoxy, phenylthio, 2-methylphenylthio, 3-methylphenylthio, 4-methylphenylthio, 2-chlorophenylthio, 3-chlorophenylthio, 4-chlorophenylthio, 2-trifluoromethylphenylthio, 2,5-dimethylphenylthio, 2,5-dichlorophenylthio, 2-chloro-5-trifluoromethylphenylthio, benzyloxy, 2-methylbenzyloxy, 3-methylbenzyloxy, 4-methylbenzyloxy, 2-chlorobenzyloxy, 3-chlorobenzyloxy, 4-chlorobenzyloxy, 2-trifluoromethylbenzyloxy, 2,5-dimethylbenzyloxy, 2,5-dichlorobenzyloxy, 2-chloro-5-trifluoromethylbenzyloxy, benzylthio, 2-methylbenzylthio, 3-methylbenzylthio, 4-methylbenzylthio 2-chlorobenzylthio, 3-chlorobenzylthio, 4-chlorobenzylthio 2-trifluoromethylbenzylthio, 2,5-dimethylbenzylthio, 2, 5-dichlorobenzylthio, 2-chloro-5-trifluoromethylbenzylthio, benzyloxyiminomethyl, 2-methylbenzyloxyiminomethyl, 3-methylbenzyloxyiminomethyl, and 4-methylbenzyloxyiminomethyl.

The heteroaromatic groups represented by A, B and D mean 5- or 6-membered heteroaromatic groups which may be substituted by one to four C₁₋₄ alkyl group(s) or halogen atom(s). The examples thereof include 2-pyridyl, 3-pyridyl, 4-pyridyl, 2-methyl-6-pyridyl, 2-furyl, 3-furyl, 3,5-dimethyl-2-furyl, 2-thienyl, 3-thienyl, 3,5-dimethyl-2-thienyl, N-methyl-3-pyrrolyl and 2,4-dimethyl-5-thiazolyl.

And, when B represents a C₁₋₂ alkylene group and D represents an optionally substituted phenyl group, B and D may form a 5- or 6-membered ring together with the phenyl group of D as shown in the following general formula (I').

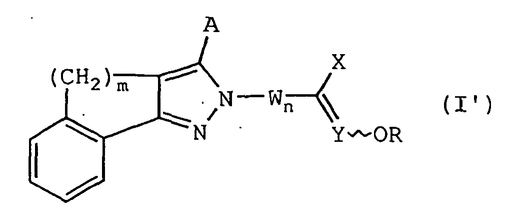

In general formula (I'), W, n, R, X and Y represent, respectively, the same as those in general formula (I); A preferably represents a hydrogen atom, a halogen atom, R¹, R¹O, R¹S, R¹SO, R¹SO₂ or a C₃₋₇ cycloalkyl group, and further preferably, a hydrogen atom or a C₁₋₄ alkyl group, m represents 1 or 2.

Next, the examples of the typical compounds of the present invention represented by general formula (I) are shown in Tables 1 to 5, and the physical properties thereof are shown in Tables 6 and 7, but are not to be construed to limit the compounds of the present invention to these examples (the compound numbers are also used in the following description) . Substituents (A, B, D, W, n, X, Y and R) in general formula (I) in page 2 are shown in the beginning in each table, respectively.

**Table 1**

| | | |
|---|---|---|
| A : as follows B : H W : CH₂ n : 1 X : COOCH₃ Y : =CH- R : CH₃ D : substituted phenyl, where the substituents are as follows | | |

| compound No | A | D(substituents) |
|---|---|---|
| 366 | H | H |
| 367 | H | 2-CH₃ |
| 368. | H | 3-C₆H₅ |
| 369 | H | 4-CN |
| 370 | H | 2-OCH₃ |
| 371 | H | 3-CF₃ |
| 372 | H | 4-OCHF₂ |
| 373 | H | 2-OCF₃ |
| 374 | H | 3-OC₆H₅ |
| 375 | H | 3-N(CH₃)₂ |
| 376 | H | 2-F |
| 377 | H | 3-Cl |
| 378 | H | 4-Br |
| 379 | CH₃ | H |
| 380 | CH₃ | 3-CH₃ |
| 381 | CH₃ | 4-C₆H₅ |
| 382 | CH₃ | 2-CN |
| 383 | CH₃ | 2-OCH₃ |
| 384 | CH₃ | 3-CF₃ |
| 385 | CH₃ | 4-OCHF₂ |
| 386 | CH₃ | 2-OCF₃ |
| 387 | CH₃ | 3-OC₆H₅ |
| 388 | CH₃ | 3-N(CH₃)₂ |
| 389 | CH₃ | 4-F |
| 390 | CH₃ | 2-Cl |
| 391 | CH₃ | 2-Br |
| 392 | CH₃ | 2,3-(CH₃)₂ |
| 393 | CH₃ | 2,5-(CH₃)₂ |
| 394 | CH₃ | 2,5-(OCH₃)₂ |
| 395 | CH₃ | 2,3-(Cl)₂ |
| 396 | CH₃ | 2,4-(Cl)₂ |
| 397 | CH₃ | 2,5-(Cl)₂ |
| 398 | CH₃ | 2,6-(Cl)₂ |
| 399 | CH₃ | 3,4-(Cl)₂ |
| 400 | CH₃ | 3,5-(Cl)₂ |

**Table 2**

| | | |
|---|---|---|
| A , R : CH₃ B : as follows W : CH₂ n : 1 X : COOCH₃ Y : =CH- D : substituted phenyl, where the substituents are as follows | | |

| compound No | B | D(substituents) |
|---|---|---|
| 401 | Cl | H |
| 402 | Cl | 3-CH₃ |
| 403 | Cl | 2-C₆H₅ |
| 404 | Cl | 4-CN , |
| 405 | Cl | 2-OCH₃ |
| 406 | Cl | 3-CF₃ |
| 407 | Cl | 4-OCHF₂ |
| 408 | Cl | 2-OCF₃ |
| 409 | Cl | 3-OC₆H₅ |
| 410 | Cl | 3-N(CH₃)₂ |
| 411 | Cl | 4-F |
| 412 | Cl | 2-Cl |
| 413 | Cl | 3-Br |
| 414 | Cl | 2,3-(CH₃)₂ |
| 415 | Cl | 2,5-(CH₃)₂ |
| 416 | Cl | 2,5-(OCH₃)₂ |
| 417 | Cl | 2,3-(Cl)₂ |
| 418 | Cl | 2,4-(Cl)₂ |
| 419 | Cl | 2,5-(Cl)₂ |
| 420 | Cl | 2,6-(Cl)₂ |
| 421 | Cl | 3,4-(Cl)₂ |
| 422 | Br | 3,5-(Cl)₂ |
| 423 | Br | 3-CH₃ |
| 424 | Br | 2-C₆H₅ |
| 425 | Br | 4-CN |
| 426 | Br | 2-OCH₃ |
| 427 | Br | 3-CF₃ |
| 428 | Br | 4-OCHF₂ |
| 429 | Br | 2-OCF₃ |
| 430 | Br | 3-OC₆H₅ |
| 431 | Br | 3-N(CH₃)₂ |
| 432 | Br | 3-F |
| 433 | Br | 2-Cl |
| 434 | Br | 4-Br |
| 435 | Br | 2,3-(CH₃) |
| 436 | Br | 2,5-(CH₃)₂ |
| 437 | Br | 2,3-(Cl)₂ |
| 438 | Br | 2,4-(Cl)₂ |
| 439 | Br | 2,5-(Cl)₂ |
| 440 | Br | 2,6-(Cl)₂ |
| 441 | Br | 3,4-(Cl)₂ |
| 442 | Br | 3,5-(Cl)₂ |
| 443 | I | H |
| 444 | I | 2-CH₃ |
| 445 | I | 3-C₆H₅ |
| 446 | I | 4-CN |
| 447 | I | 2-OCH₃ |
| 448 | I | 3-CF₃ |
| 449 | I | 4-OCHF₂ |
| 450 | I | 2-OCF₃ |
| 451 | I | 3-OC₆H₅ |
| 452 | I | 3-N(CH₃)₂ |
| 453 | I | 2-F |
| 454 | I | 3-Cl |
| 455 | I | 4-Br |
| 456 | CH₃ | H |
| 457 | CH₃ | 2-CH₃ |
| 458 | CH₃ | 3-C₆H₅ |
| 459 | CH₃ | 4-CN |
| 460 | CH₃ | 2-OCH₃ |
| 461 | CH₃ | 3-CF₃ |
| 462 | CH₃ | 4-OCHF₂ |
| 463 | CH₃ | 2-OCF₃ |
| 464 | CH₃ | 3-OC₆H₅ |
| 465 | CH₃ | 3-N(CH₃)₂ |
| 466 | CH₃ | 2-F |
| 467 | CH₃ | 3-Cl |
| 468 | CH₃ | 4-Cl |
| 469 | CH₃ | 4-Br |
| 470 | CN | 3-Cl |
| 471 | CH=NOCH₃ | 3-Cl |
| 472 | CH₂CH=CH₂ | 3-Cl |
| 473 | CH₂OCH₃ | 3-Cl |
| 474 | CO₂CH₃ | 3-Cl |
| 475 | NO₂ | H |
| 476 | SCH₃ | H |

**Table 3**

| | | | |
|---|---|---|---|
| A, B : as follows W : CH₂ n : 1 X : COOCH₃ Y : =CH- R : CH₃ D : substituted phenyl, where the substituents are as follows | | | |

| compound No | A | D(substituents) | B |
|---|---|---|---|
| 477 | CF₃ | 2-Cl | H |
| 478 | C₂H₃ | H | H |
| 479 | c-C₃H₅ | 3-Cl | H |
| 480 | i-C₃H₇ | 3-Cl | H |
| 481 | t-C₄H₉ | 3-Cl | H |
| 482 | SCH₃ | H | H |
| 483 | SOCH₃ | H | H |
| 484 | SO₂CH₃ | H | H |
| 485 | SCH₃ | 3-Cl | H |
| 486 | SOCH₃ | 3-Cl | H |
| 487 | SO₂CH₃ | 3-Cl | H |
| 488 | SCH₃ | 4-Br | H |
| 489 | CF₃ | 2-Cl | Cl |
| 490 | C₂H₃ | H | Cl |
| 491 | c-C₃H₅ | 3-Cl | Cl |
| 492 | i-C₃H₇ | 3-Cl | Cl |
| 493 | t-C₄H₉ | 3-Cl | Cl |
| 494 | SCH₃ | H | Cl |
| 495 | SOCH₃ | H | Cl |
| 496 | SO₂CH₃ | H | Cl |
| 497 | SCH₃ | 3-Cl | Cl |
| 498 | SOCH₃ | 3-Cl | Cl |
| 499 | SO₂CH₃ | 3-Cl | Cl |
| 500 | SCH₃ | 4-Br | Cl |
| 501 | CF₃ | 2-Cl | Br |
| 502 | C₂H₃ | H | Br |
| 503 | c-C₃H₅ | 3-Cl | Br |
| 504 | i-C₃H₇ | 3-Cl | Br |
| 505 | t-C₄H₉ | 3-Cl | Br |
| 506 | SCH₃ | H | Br |
| 507 | SOCH₃ | H | Br |
| 508 | SO₂CH₃ | H | Br |
| 509 | SCH₃ | 3-C1 | Br |
| 510 | SOCH₃ | 3-Cl | Br |
| 511 | SO₂CH₃ | 3-C1 | Br |
| 512 | SCH₃ | 4-Br | Br |
| 513 | Cl | H | CH₃ |
| 514 | Br | H | CH₃ |

**Table 4**

| | |
|---|---|
| A , R : CH₃ B : as follows D : C₆H₅ W : CH₂ n : 1 X : CONHCH₃ Y : =CH- | |

| compound No | B |
|---|---|
| 601 | H |
| 602 | Cl |
| 603 | Br |
| 604 | I |

**Table 5**

| | | | | | |
|---|---|---|---|---|---|
| A : CH₃ X : COOCH₃ B, W, n, Y, R : as follows D : substituted phenyl, where the substituent is 3-Cl | | | | | |

| compound No | B | W | n | Y | R |
|---|---|---|---|---|---|
| 702 | H | CH₂ | 1 | CH | FCH₂ |
| 703 | Cl | CH₂ | 1 | CH | FCH₂ |
| 704 | Br | CH₂ | 1 | CH | FCH₂ |
| 705 | I | CH₂ | 1 | CH | FCH₂ |
| 714 | H | CH₂ | 1 | CH | F₂CH |
| 715 | Cl | CH₂ | 1 | CH | F₂CH |
| 716 | Br | CH₂ | 1 | CH | F₂CH |
| 717 | I | CH₂ | 1 | CH | F₂CH |

**Table 6**

| compound No | physical state (mp:melting point) |
|---|---|
| 379 | oil |
| 380 | mp68-70°C |
| 390 | mp91-92°C |
| 393 | oil |
| 395 | oil |
| 396 | mp97-99°C |
| 397 | oil |
| 398 | mp121-123°C |
| 399 | mp74-76°C |
| 400 | mp114-116°C |
| 401 | mp69-71°C |
| 402 | mp70-72°C |
| 412 | oil |
| 415 | oil |
| 416 | oil |
| 417 | oil |
| 418 | oil |
| 419 | oiol |
| 420 | mpl07-108°C |
| 421 | mp115-117°C |
| 422 | mp98-100°C |
| 423 | oil |
| 433 | oil |
| 436 | oil |
| 437 | oil |
| 438 | oil |
| 439 | oil |
| 441 | mp142-145°C |
| 442 | mp102-105°C |
| 456 | oil |
| 467 | mp98-100°C |
| 468 | oil |
| 471 | mp134-136°C |
| 478 | oil |
| 479 | oil |
| 482 | oil |
| 485 | oil |
| 490 | oil |
| 491 | oil |
| 494 | oil |
| 497 | oil |
| 502 | oil |
| 506 | oil |
| 513 | resin |
| 514 | resin |
| 601 | oil |

**Table 7**

| No. | δ (ppm) solvent: chloroform-d internal standard substance : tetramethylsilane |
|---|---|
| 379 | 2.36(3H,s), 3.69(3H,s), 3.91(3H,s), 4.88(2H,s), 6.23 (1H,s), 7.10-7.90(6H,m) |
| 393 | 2.29(3H,s), 2.37(3H,s), 2.39(3H,s), 3.70(3H,s), 3.90 (3H,s), 4.89 (2H, s), 6.08 (1H, s), 6.80-7.60 (4H,m) |
| 395 | 2.39(3H,s), 3.69(3H,s), 3.89(3H,s), 4.90(2H,s), 6.47 (1H,s), 7.00-7.80(4H,m) |
| 397 | 2.39(3H,s), 3.70(3H,s), 3.92(3H,s), 4.90(2H,s), 6.51 (1H,s), 7.00-7.90(4H,m) |
| 412 | 2.10(3H,s), 2.27(3H,s), 3.76(3H,s), 3.83(3H,s), 4.80 (2H,s), 7.10-7.80(6H,m) |
| 415 | 2.24(3H,s), 2.30(3H,s), 2.35(3H,s), 3.70(3H,s), 3.89 (3H,s), 4.90(2H,s), 6.80-7.60 (4H,m) |
| 416 | 2.33 (3H,s), 3.66(3H,s), 3.86(3H,s), 4.87(2H,s), 7.00-7.65(4H,m) |
| 417 | 2.36(3H,s), 3.70(3H,s), 3.90(3H,s), 4.90(2H,s), 7.10-7.60(4H,m) |
| 418 | 2.36(3H,s), 3.70(3H,s), 3.90(3H,s), 4.90(2H,s), 7.10-7.60(4H,m) |
| 419 | 2.33(3H,s), 3.64(3H,s), 3.38(3H,s), 4.90(2H,s), 7.17-7.41(4H,m) |
| 423 | 2.47(6H,s), 3.67(3H,s), 3.86(3H,s), 4.88(2H,s), 7.00-7.80(4H,m) |
| 433 | 2.16(3H,s), 2,38(3H,s), 3.70(3H,s), 3.89(3H,s), 4.95 (2H,s), 7.05-7.60(5H,m) |
| 436 | 2.22(3H,s), 2,30(3H,s), 2.37(3H,s), 3.70(3H,s), 3.89 (3H,s), 4.92(2H,s), 6.90-7.60(4H,m) |
| 437 | 2.35(3H,s), 3.66(3H,s), 3.84(3H,s), 4.90(2H,s), 7.05-7.60(4H,m) |
| 438 | 2.37(3H,s), 3.70(3H,s), 3.89(3H,s), 4.93(2H,s), 7.10-7.70(4H,m) |
| 439 | 2.38(3H,s), 3.70(3H,s), 3.90(3H,s), 4.94(2H,s), 7.10-7.60(4H,m) |
| 456 | 2.10(3H,s), 2.27(3H,s), 3.70(3H,s), 3.90(3H,s), 4.88 (2H,s), 7.00-7.80(6H,m) |
| 468 | 2.05(3H,s), 2.25(3H,s), 3.65(3H,s), 3.86(3H,s), 4.83 (2H,s), 7.10-7.70(5H,m) |
| 478 | 1.30(3H,t,J=7.5Hz), 2.75(2H,q,J=7.5Hz), 3.68(3H,s), 3.89(3H,s), 4.88(2H,s), 6.27(1H,s), 7.00-7.90(6H,m) |
| 479 | 0.67-1.65(5H,m), 3.69(3H,s), 3.89(3H,s), 5.03(2H,s), 6.06(1H,s), 7.17-7.72(6H,m) |
| 482 | 2.48(3H,s), 3.71(3H,s), 3.90(3H,s), 5.07(2H,s), 6.55 (1H,s), 7.10-7.90(6H,m) |
| 485 | 2.47(3H,s), 3.71(3H,s), 3.92(3H,s), 5.06(2H,s), 6.52 (1H,s), 7.10-7.80(5H,m) |
| 490 | 1.21(3H,t,J=7.7Hz), 2.81(2H,q,J=7.7Hz), 3.64(3H,s), 3.84(3H,s), 4.85(2H,s), 7.10-8.00(6H,m) |
| 491 | 0.89-1.95(5H,m), 3.69(3H,s), 3.89(3H,s), 5.03(2H,s), 7.17-7.40(2H,m), 7.54(1H,s), 7.64-7.81(2H,m) |
| 494 | 2.41(3H,s), 3.68(3H,s), 3.87(3H,s), 5.13(2H,s), 7.20-8.00(6H,m) |
| 497 | 2.43(3H,s), 3.70(3H,s), 3.91(3H,s), 5.15(2H,s), 7.20-8.00(5H,m) |
| 502 | 1.19(3H,t,J=7.6Hz), 2.82(2H,q,J=7.6Hz), 3.65(3H,s), 3.82(3H,s), 4.87(2H,s), 7.10-8.00(6H,m) |
| 506 | 2.42(3H,s), 3.70(3H,s), 3.88(3H,s), 5.18(2H,s), 7.20-8.00(6H,m) |
| 513 | 1.89(3H,s), 3.70 (3H,s), 3.88 (3H,s), 4.64 (2H,s), 7.10-8.10(6H,m) |
| 514 | 2.15 (3H,s), 3.75 (3H,s), 3.97 (3H,s), 5.29 (2H,s), 7.10-8.00(6H,m) |
| 601 | 2.34 (3H, s), 2.80-3.10 (3H,m), 3.68 (3H, s), 4.98 (2H, s), 6.25 (1H, s), 7.00-8.00(6H, m) |

### [Preparation]

### Preparation 1:

Among the compounds of the present invention represented by general formula (I), the compound (Ia) wherein n is 1, W represents a C₁₋₃ alkylene group, X represents R¹OOC-, and Y represents CH may be prepared, according to the following reaction scheme (A), by allowing the alkanoate derivative represented by general formula (III) to react with a formylating agent in the presence of base and a solvent to give the compound represented by general formula (II) (process (a)), and then allowing it to react with an alkylating agent in the presence of base (process (b)).

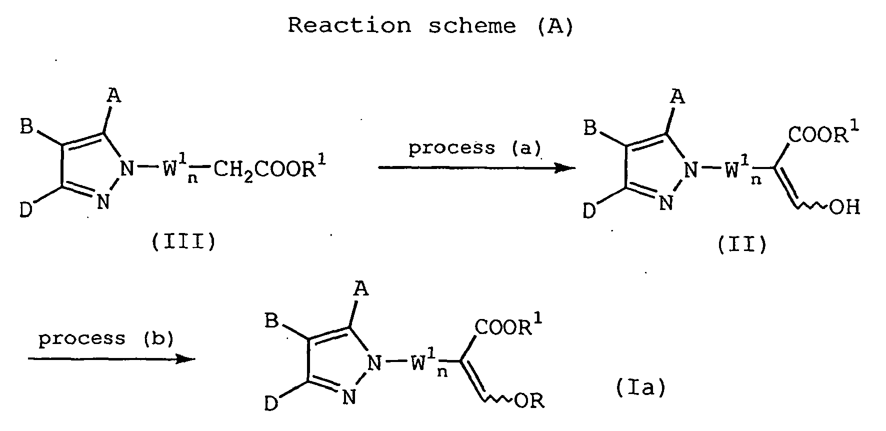

In the reaction scheme (A), W¹ represents a C₁₋₃ alkylene group and the other symbols represent the same as the above, respectively.

### Process (a)

In the reaction of the alkanoate derivative represented by general formula (III) with the formylating agent, the formylating agent used includes formates such as methyl formate, and acid amides such as N,N-dimethylformamide.

The base used includes: alkali metals and the hydrides thereof such as potassium metal, potassium hydride, sodium metal, and sodium hydride; metal alcoholates such as potassium tert-butoxide and sodium methylate; alkyllithium or aryllithium such as butyllithium and phenyllithium; and, lithium amides such as N,N-diisopropyllithium amide.

With respect to the amounts of the reagents subjected to the reaction, about 1 to 10 equivalents of the formylating agent and about 1 to 3 equivalents of the base are used to 1 equivalent of the alkanoate derivative represented by general formula (III).

The reaction is usually carried out under atmospheric pressure. The reaction temperature is usually from -70 to 100 °C, and the reaction time is from 5 minutes to 24 hours.

In the process (a), a solvent is usually used. The solvent used includes: ethers such as diethyl ether, 1,2-dimethoxyethane, tetrahydrofuran and 1,4-dioxane; and, acid amides such as N,N-dimethylformamide, N-methyl-5-pyrrolidone and N,N-dimethylacetamide.

After the reaction, the reaction mixture is acidified with acids such as hydrochloric acid and sulfuric acid, and post-treated by a conventional method, and purified by chromatography, recrystallization, distillation and the like, if needed, to obtain the compound represented by general formula (II).

### Process (b)

In the reaction of the compound represented by general formula (II) with the alkylating agent in the process (b), the alkylating agent used includes alkyl halides such as methyl iodide, and sulfate esters such as dimethyl sulfate.

The base used includes tertiary amines such as pyridine, triethylamine, 4-N,N-dimethylaminopyridine, 1,8-diazabicyclo[5.4.0]undecene-7, and 1,4-diazabicyclo[2.2.2]octane; alkali metals and the hydrides thereof such as potassium metal, potassium hydride, sodium metal, and sodium hydride; metal alcoholates such as potassium tert-butoxide and sodium methylate; and, carbonates such as potassium carbonate and sodium carbonate.

With respect to the amounts of the reagents subjected to the reaction, about 1 to 5 equivalents of the alkylating agent and about 1 to 5 equivalents of the base are used to 1 equivalent of the compound represented by general formula (II).

In the process (b), a solvent is usually used. The solvent used includes: ethers such as diethyl ether, 1,2-dimethoxyethane, tetrahydrofuran and 1,4-dioxane; acid amides such as N,N-dimethylformamide, N-methyl-5-pyrrolidone and N,N-dimethylacetamide; and, ketones such as acetone and methyl ethyl ketone.

The reaction described above is usually carried out under atmospheric pressure. The reaction temperature is usually from -70 to 100°C, and the reaction time is from 5 minutes to 24 hours.

After the reaction, the reaction mixture may be post-treated by a conventional method, and purified by chromatography, recrystallization, distillation and the like, if needed, to obtain the desired compound of the present invention represented by general formula (Ia).

In the present invention, the compound represented by general formula (Ia) may be prepared also from the alkanoate derivative represented by general formula (III) in a pot without isolating the compound represented by general formula (II).

That is, the compound of the present invention represented by general formula (Ia) may be prepared by allowing the alkanoate derivative represented by general formula (III) to react with a formate using the above-mentioned base and solvent to be led to the compound represented by general formula (II) in the reaction system, followed by allowing it to react with an alkylating agent such as alkyl halides and sulfate esters.

And especially, the compound wherein B represents a halogen atom is desirably prepared by halogenating using a halogenating agent the compound wherein B represents a hydrogen atom which represents previously prepared.

### Preparation 2:

Among the compounds of the present invention represented by general formula (I), the acrylamide derivative (Ib) wherein n is 1, W represents a C₁₋₃ alkylene group, X represents R¹HNOC- or R¹R¹NOC- (in these groups, R¹ represents the same as the above, and the two R¹s in R¹R¹NOC- may be same or different.) and Y represents CH may be prepared, according to the following reaction scheme (B), by allowing the acrylate derivative represented by general formula (Ia) which is obtained by the above-mentioned preparation 1 to react with an alkylamine (HNR¹R¹) in a proper solvent.

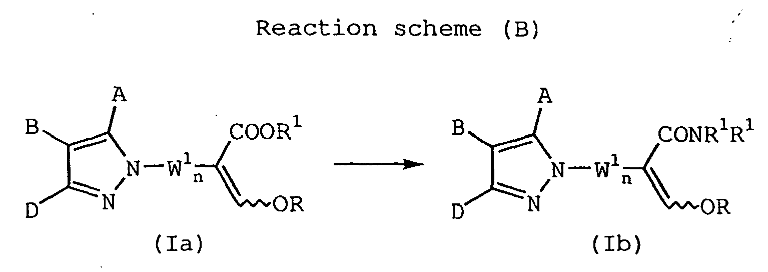

The symbols in this reaction scheme represent the same as the above.

The reaction above is usually carried out under atmospheric pressure, the reaction temperature is usually from 0 to 150 °C, and the reaction time is in the range of 5 minutes to 24 hours.

The solvent which may be used includes: alcohols such as ethanol and methanol; ethers such as diethyl ether, 1,2-dimethoxyethane, tetrahydrofuran and 1,4-dioxane; and acid amides such as N,N-dimethylformamide, N-methyl-5-pyrrolidone and N,N-dimethylacetamide.

The material alkanoate derivative represented by general formula (III) may be prepared, according to the following reaction scheme, by allowing the pyrazole derivative represented by general formula (V) to react with the haloalkanoate represented by general formula (IV) in the presence of base.

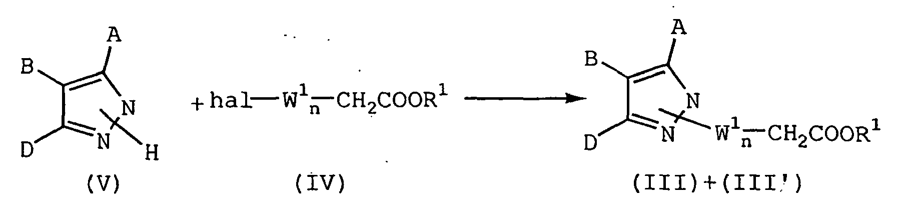

In this reaction scheme, hal represents a halogen atom, and the other symbols represent the same as the above.

In the reaction above, the base used includes: tertiary amines such as pyridine, triethylamine, 4-N,N-dimethylaminopyridine, 1,8-diazabicyclo[5.4.0]undecene-7, and 1,4-diazabicyclo[2.2.2]octane; metal alcoholates such as potassium tert-butoxide and sodium methylate; and, inorganic salts such as lithium hydroxide, potassium hydroxide, sodium hydroxide, sodium carbonate, potassium carbonate, and sodium hydride.

The solvent which may be used includes: ethers such as diethyl ether, 1,2-dimethoxyethane, tetrahydrofuran and 1,4-dioxane; acid amides such as N,N-dimethylformamide, N-methyl-5-pyrrolidone and N,N-dimethylacetamide; hydrocarbons such as benzene and toluene; and, acetonitrile and the like.

The reaction is usually carried out under atmospheric pressure, the reaction temperature is usually from -70 to 150 °C, and the reaction time is from 10 minutes to 48 hours.

In the reaction above, addition of 1 to 20 mole% of crown ethers such as 18-crown-6 or 15-crown-5 to the substrate represented by general formula (V) to the reaction system may exert the effects to shorten the reaction time and increase the yield of the desired product in certain cases.

With respect to the amounts of the reagents used in the reaction, 1 to 10 equivalents of the alkanoate derivative represented by general formula (IV) and 1 to 10 equivalents of the base are used to 1 equivalent of the pyrazole derivative represented by general formula (V).

After the reaction, the reaction mixture may be post-treated by a conventional method, and purified by chromatography, recrystallization and the like, if needed, to obtain the desired product. And the compound represented by general formula (III) and that represented by general formula (III') which is the positional isomer thereof may be isolated, or may be used in the reaction of the reaction scheme (A) above without isolation and purified in a final step.

The pyrazole derivative represented by general formula (V) which is used as the starting material in the process above may be prepared, e.g, according to the following reaction scheme.

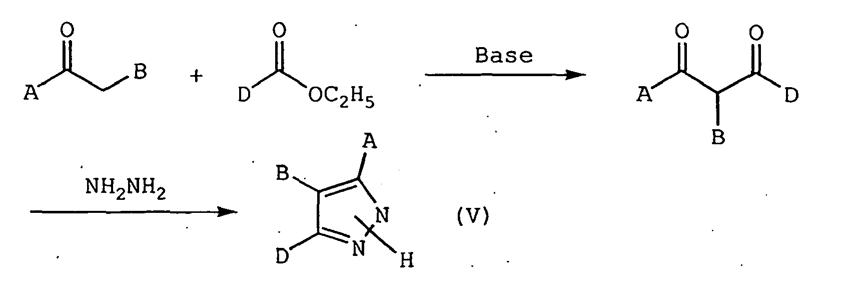

The symbols in this scheme represent the same as the above.

And especially, the compound wherein A or D represents a phenyl group substituted by plural fluorine atoms or fluorine-containing groups may be prepared by the reaction using samarium metal and iodine (*Synthetic Communications,* **26**(12), 2412-2427 (1996)), or the reaction using magnesium chloride (*J. Org. Chem.,* **50,** 2622-2624 (1985)) in the first process, and acidification of the reaction system by adding acid (Japanese Patent Application Laid-Open No. JP95-53439).

### [Fungicides]

The compounds represented by general formula (I) have fungicidal activity and are used as fungicides in agriculture and horticulture and for industrial materials.

### Application as fungicides in agriculture and horticulture

The compounds represented by general formula (I) (objective compounds) may be applied as fungicides by treating plants with the active ingredients through spraying, dispersing, spreading and the like, or by treating seeds of plants, soil around plants, or soil which is sowed with seeds, paddy fields or water in water culture with the active ingredients. The application may be made before or after infection with disease-causing germs.

The objective compounds may be used in the common formulations suitable for agricultural fungicidal agents such as liquids, wettable powders, emulsions, suspensions, liquid concentrates, tablets, granules, aerosols, pastes, powders and smokes. Such formulations may be obtained by an ordinary method of blending at least one kind of the compounds of the present invention with proper solid or liquid carriers, and, if desired, proper adjuvants (e.g., surface-active agents, spreaders, dispersers, and stabilizers) to improve dispersibility and other properties of the active ingredients.

The examples of the solid carriers or diluents include plant substances, fibrous substances, artificial plastic powders, clays (e.g., kaolin, bentonite, and white clay), talc and inorganic matters (pumice, sulfur powders), and chemical fertilizers. The examples of the liquid carriers or diluents include water, alcohols, ketones, ethers, aromatic hydrocarbons, aliphatic hydrocarbons, esters, nitriles, amides (N,N-dimethylformamide, dimethylsulfoxide), and hydrocarbon halogenides.

The examples of the surface-active agents include alkyl sulfate, alkyl sulfonate, polyethylene glycol ether, and polyhydric alcohol esters. The examples of the spreaders or dispersers include casein, gelatin, starch powders, carboxymethylcellulose, gum arabic, alginic acid, lignin, bentonite, polyvinylalcohol, pineapple oil, honey, agar, and the like.

The stabilizers include isopropylphosphate mixtures, tricresyl phosphate, tall oil, epoxy oil, surface-active agents, fatty acids and the esters thereof. In addition to the above-mentioned ingredients, the formulations of the present invention may be used in combination with other fungicides, insecticides, herbicides, or fertilizers.

Generally, the formulations above contain 1 to 95 wt.%, preferably 1 to 50 wt.% of at least one kind of the objective compounds. These formulations may be used alone or with dilution, and the objective compounds may be used in amount of about from 1 g to 5 kg/hectare, preferably about from 2 g to 100 g/hectare, and in concentration of usually about from 1 to 50,000 ppm, preferably about from 50 to 1,000 ppm.

The plant diseases that the compounds of the present invention represented by general formula (I) exert excellent effects to control include: *Pyricularia oryzae, Cochliobolus miyabeanus, Rhizoctonia solani,* and *Gibberella fujikuroi* on rice plant; *Erysiphe graminis f.sp. hordei, Erysiphe graminis f.sp. tritici, Puccinia striiformis, Puccinia graminis, Puccinia recondita f. sp. tritici, Puccinia hordei, Gibberella zeae, Pyrenophora teres, Typhula incarnata, Typhula ishikariensis, Sclerotinia borealis, Micronectriell a nivalis, Ustilago nuda, Tilletia caries, Tilletia toetida, Tapesia yallundea, Phynchosporium secalis f.sp. hordei, Septoria tritici,* and *Lentosphaeria nodorum* on wheat or barley plants; *Diaporthe citri, Elsinoe fawcettii, Phytophthora citrophthora, Penicillium digitatum,* and *Penicillium italicum* on citrus fruits; *Monilinia mali, Valsa ceratosperma, Podosphaera leucotricha, Alternaria alternata apple pathotype, Venturia inaequalis, Gymnosporangium yamadae, Botriophaeria berengeriana f. sp. piricola, Zygophiala jamaicensis, Gloeodes pomigena, Mycosphaerella pomi, Glomerella cingulata,* and *Diplocarpon mali* on apples; *Venturia nashicola, Alternaria alternata japanese pear pathotype, Physalospora piricola,* and *Gymnosporangium asiaticum* on pears; *Monilinia fructicola, Cladosporium carpophilum,* and *Phomopsis sp.* on peaches; *Plasmopara viticola, Pseudocercospora vitis, Marssonina viticola, Elsinoe ampelina, Glomerella cingulata, Uncinula necator, Phakopsora ampelopsidis,* and *Phomopsis sp.* on grapes; *Phyllactinia kakicola, Colletotrichum gloeosporioides, Cercospora kaki*, and *Mycosphaerella n* awae on persimmons; *Cladosporium carpophilum* on Japanese apricots; *Monilinia fructicola* on cherries; *Sphaerotheca fuliginea, Pseudoperonospora cubensis, Didymella bryoniae, Colletotorichum legenarium, Pythium cucurbitacearum, Pythium debaryanum, Rhizoctonia solani, Alternaria solani, Cladosporium fulvum,* and *Phytophthora infestans* on the gourd family; *Phomopsis vexans,* and *Erysiphe cichoracearum* on eggplants; *Alternaria ja ponica, Alternaria bracicae, Alternaria brassicicola,* and *Cercosporella brassicae* on cruciferous vegetables; *Peronospora spinaciae,* and *Phytophthora sp.* on spinach; *Peronospora destructor, Phytophthora nicotiana,* and *Botrytis allii* on onions; *Puccinia allii* on spring onions; *Pyrhium ultimum,* and *Pythium zigiberis* on gingers; *Sphaerotheca humuli,* and *Glomerella cingulata* on strawberries; *Cercospora kikuchii, Peronospora manshurica, Elsinoe glycines,* and *Diaporthe phaseolorum var. sojae* on soybeans; *Cercospora canescens,* and *Uromyces phaseoli var. azukicola* on adzuki beans; *Colletotrichum lindemuthianum* on kidney beans; *Cercosporidium personatum, Cercospora arachidicola,* and *Shaceloma arachidis* on peanuts; *Erysiphe pisi* on peas; *Alternaria solani,* and *Phytophthora infestans* on potatoes; *Exobasidium reticulatum, Elsinoe leucospila, Pestalotiopsis theae,* and *Pestalotiopsis longiseta* on tea plants; *Alternaria longipes, Erysiphe cichoracearum,* and *Colletotrichum gloeosporioides* on tobaccos; *Cercospora beticola* on beets; *Curvularia geniculata, Rhizoctonia solani, Ceratobasidium spp. , Pythium periplocum, Pythium graminicola,* and *Pythium vanterpoolii* on brushwood; *Diplocarpon rosae,* and *Shaerotheca pannosa* on roses; *Septoria obesa,* and *Puccinia horiana* on chrysanthemums; and, *Botrytis cinerea,* and *Sclerotinia sclerotiorum* on various crops and the like.

### Application as fungicides in industrial use

The compounds according to the present invention represented by general formula (I) may be applied also as fungicides for protection of industrial materials. When the compounds (I) according to the present invention is blended into the industrial materials exposed to the attack by microorganisms, the growth of the microorganisms will be inhibited to maintain the inherent value of the materials for a long term.

The industrial materials to which the fungicides of the present invention may be applied are the products prepared from natural or synthetic materials but not existing in nature, and are subjected to degradation or denaturation by microorganisms. The examples include adhesives, glue, paper and thick paper, fibrous materials, leather, wood, coating materials, plaster, lubricating oil for cooling, and plastic products. And also, the fungicides of the present invention may be applied to walls of water supply for cooling and lubricating oil for cooling in manufacturing plants. Among them, the fungicides may be used preferably for adhesives, paper, thick paper, coating materials, wood, and the like.

The microorganisms that may cause degradation and denaturation of the industrial materials include bacteria, fungi, yeast, algae and microorganisms of slime, and the compounds of the present invention are preferentially active against fungi and microorganisms of slime. And also, they have excellent fungicidal action on mold, wood degradative fungi, and wood discoloring fungi.

The microorganisms on which the compounds of the present invention exert excellent fungicidal effects include Alternaria such as *Alternaria tenuis,* and *Alternaria speciales;* Aspergillus such as *Aspergillus niger,* and *Aspergillus flavus;* Chaetomium such as *Chaetomium globosum;* Conjophora such as *Conjophora cerebella*; Lentinus such as *Lentinus tigrinus;* Penicillium such as *Penicillium glaucum,* and *Penicillium citrium*; Polyporus such as *Polyporus versicola;* Aureobasidium such as *Aureobasidium pullulans;* Schlerophoma such as *Schlerophoma pityophia;* Stachybotrys such as *Stachybotrys atra;* Paecilomyces such as *Paecilomyces varioti;* and, Cladosporium such as *Cladosporium herbarum.*

The compounds of the present invention may be used in the formulations suitable for the application field of fungicides in industrial use such as liquids, emulsions, granules, pastes and powders. Such formulations may be prepared, for instance, by a known method of blending at least one kind of the compounds of the present invention with bulking agents such as liquid solvents and/or solid carriers. And, if desired, surface-active agents such as emulsifiers and/or dispersers may be contained. When water is used as the bulking agent, organic solvents may be used as adjunctive solvents.

The solvents which may be used include alcohols such as lower alcohols, preferably ethanol and 2-propanol, and benzyl alcohol; ketones such as acetone and methyl ethyl ketone; liquid hydrocarbons such as petroleum fractions; and, hydrocarbon chlorides such as 1,2-dichloroethane.

The fungicides in industrial use according to the present invention contain generally 10 to 100 wt.%, preferably 10 to 50 wt.% of the compounds of the present invention.

The concentration used of the present compounds depend upon the properties and degrees of the microorganisms to be controlled, and the composition of the materials to be protected, but is generally in the range of 0.001 to 5 wt.%, preferably 0.5 to 1.0 wt.% to the materials to be protected.

The fungicides of the present invention may be blended with other known ingredients of fungicides in addition to the compounds according to the present invention in order to extent the spectrum of the action.

The examples of the other ingredients of fungicides include bactericides, phenol derivatives, compounds eliminating formaldehyde, dithiocarbamate, benzimidazolylcarbamate, thioazolylbenzimidazole, isothiazolone derivatives, benzoisothiazolone derivatives, trihalomethylthio compounds, dinitrile tetrachloroisophthalate, mercaptobenzothiazole, and mercaptopyridine.

### BEST MODE FOR CARRYING OUT THE INVENTION

In the following, descriptions of examination examples of the compounds of the present invention are provided, but are not to be construed to limit the invention to the examples.

### Test example

In the following, descriptions of the test examples of the present invention as fungicides in agriculture and horticulture are provided.

The preventive value is estimated of each tests. For this purpose, the ratio of lesion area or the length of lesion or the number of lesions on plants are observed with the naked eye. The preventive value of each compounds is given by the ratio 100(N-n)/N where N is the ratio of lesion area or the length of lesion or the number of lesions on untreated plants and n is that of treated plants.

The control effect is shown as the control index comprising the following 6 grades. The judgement was made according to the present judgement criterion unless otherwise noted.

| | |
|---|---|
| Index 5: | the preventive value is 100 %. |
| Index 4: | the preventive value is from 90 to 99 %. |
| Index 3: | the preventive value is from 70 to 89 %. |
| Index 2: | the preventive value is from 50 to 69 %. |
| Index 1: | the preventive value is from 1 to 49 %. |
| Index 0: | the preventive value is 0 %. |

### Examination experiment 1: Controlling effect against Sphaerotheca fuliginea on cucumber

Over the cucumbers (variety: Hikari No.3, type P) in the 3-leaf period cultivated in plastic pots of 6 cm diameter, the conidia of *Sphaerotheca fuliginea* on cucumbers were sprinkled to inoculate. After 3 days, the corresponding amount to 200 liter/10 a of the preparation solution obtained by adjusting the concentration of the compound of the active ingredient to 500 ppm by dilution with water containing Tween 20 was treated using a spray gun. After that, the pots were maintained in a greenhouse, and 12 days after the inoculation, the areas of disease lesion on the first and second leaves were examined to obtain the control index according to the judgement criterion above. As a result, the following compounds exhibited Index 5 at 500 ppm.

Nos. of the compounds of the present invention exhibiting Index 5: 380, 395, 396, 397, 399, 400, 401, 402, 416, 418, 420, 421, 422, 423, 437, 439, 442, 467, 468, 479, 490, 491, 502.

### Examination experiment 2: Controlling effect against Pyricularia oryzae on rice

Over the rice (variety: Koshihikari) in the 3-leaf period cultivated in plastic pots of 9 cm diameter, the corresponding amount to 200 liter/10 a of the preparation solution obtained by adjusting the concentration of the compound of the active ingredient to 500 ppm by dilution with water containing Tween 20 was treated using a spray gun. The next day, the suspension of the conidia of *Pyricularia oryzae* were sprayed to inoculate. The pots were maintained in a moist chamber at 23 °C for 24 hours and in a greenhouse for 7 days, and the number of the disease lesions on the inoculated blades was examined to obtain the control index according to the judgement criterion above. As a result, the following compounds exhibited Index 5 at 500 ppm.

Nos. of the compounds of the present invention exhibiting Index 5: 379, 380, 393, 395, 397, 399, 401, 402, 422, 423, 437, 439, 442, 456, 467, 479, 482, 485, 491, 497, 502, 513.

### Examination experiment 3: Controlling effect against Pseudoperonospora cubensis on cucumber

Over the cucumbers (variety: Hikari No.3, type P) in the 3-leaf period cultivated in plastic pots of 6 cm diameter, the corresponding amount to 200 liter/10 a of the preparation solution obtained by adjusting the concentration of the compound of the active ingredient to 500 ppm by dilution with water containing Tween 20 was treated using a spray gun. The next day, the suspension of the zoospores of *Pseudoperonospora cubensis* were sprayed to inoculate. The pots were maintained in a moist chamber at 22 °C for 18 hours and in a greenhouse for 5 days, and the area of lesion was examined to obtain the control index according to the judgement criterion above. As a result, the following compounds exhibited Index 5 at 500 ppm.

Nos. of the compounds of the present invention exhibiting Index 5: 379, 380, 396, 397, 399, 400, 401, 402, 420, 421, 422, 438, 441, 442, 456, 467, 468, 478, 482, 485, 490, 491, 494, 502, 506.

### Examination experiment 4: Controlling effect against Rhizoctonia solani on rice

Over the rice (variety: Koshihikari) in the 3-leaf period cultivated in plastic pots of 9 cm diameter, the corresponding amount to 200 liter/10 a of the preparation solution obtained by adjusting the concentration of the compound of the active ingredient to 500 ppm by dilution with water containing Tween 20 was treated using a spray gun. The next day, the mycelial colony of *Rhizoctonia solani* cultivated on PDA medium was punched by a cork borer and patched on the sheaths to inoculate. The pots were maintained in a moist chamber at 28 °C for 24 hours and in a greenhouse for 3 days, and the length of the disease lesion was examined to obtain the control index according to the judgement criterion above. As a result, the following compounds exhibited Index 5 at 500 ppm.

Nos. of the compounds of the present invention exhibiting Index 5: 380, 390, 395, 397, 399, 401, 402, 412, 415, 416, 417, 418, 420, 421, 422, 423, 433, 436, 437, 438, 439, 442, 456, 467, 468, 479, 490, 497.

### Examination experiment 5: Controlling effect against Alternaria brassicicola on Chinese cabbages

Over the Chinese cabbages (variety: Nozaki No.2) in the 5-leaf period cultivated in plastic pots of 6 cm diameter, the corresponding amount to 200 liter/10 a of the preparation solution obtained by adjusting the concentration of the compound of the active ingredient to 500 ppm by dilution with water containing Tween 20 was treated using a spray gun. The next day, the suspension of the conidia of *Alternaria brassicicola* were sprayed to inoculate. The pots were maintained in a moist chamber at 24 °C for 3 days and in a greenhouse for a day, and the area of lesion on the inoculated leaves was examined to obtain the control index according to the judgement criterion above. As a result, the following compounds exhibited Index 5 at 500 ppm.

Nos. of the compounds of the present invention exhibiting Index 5: 390, 393, 412, 415, 416, 433, 437, 468.

### Examination experiment 6: Controlling effect against Botrytis cinerea on cucumbers

Over the cucumbers (variety: Hikari No.3, type P) in the cotyledonal period cultivated in plastic pots of 9 cm diameter, the corresponding amount to 200 liter/10 a of the preparation solution obtained by adjusting the concentration of the compound of the active ingredient to 500 ppm by dilution with water containing Tween 20 was treated using a spray gun. The next day, the suspension of the conidia of *Botrytis cinerea* of cucumbers were sprayed to inoculate. The pots were maintained in a moist chamber at 22 °C for 24 hours and in a greenhouse for 2 days, and the area of lesion was examined to obtain the control index according to the judgement criterion above. As a result, the following compounds exhibited Index 5 at 500 ppm.

Nos. of the compounds of the present invention exhibiting Index 5: 379, 380, 390, 395, 401, 412, 422, 423, 433, 456, 467.

### Industrial Applicability

The novel substituted pyrazole derivatives represented by general formula (I) have excellent penetration and translamina properties and exert the control effect (prevention and curative) on damage of various diseases of plants, but are safe for crops, and therefore, are useful as fungicides in agriculture and horticulture.

Also, they are useful as fungicides for industrial materials such as adhesives, glue, paper and thick paper, fibrous materials, leather, wood, coating materials, plaster, lubricating oil for cooling, and plastic products.

## Claims

1. A compound having following general formula (I): 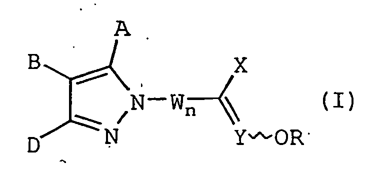 wherein
X represents R¹OOC, R¹HNOC, R¹R¹NOC, CN or a 5- or 6-membered heteroaromatic group;
Y represents CH or N;
W represents C₁₋₃ alkylene, NR¹, or O;
n is 1;
R represents C₁₋₄ lower alkyl which may be optionally substituted by one or more same or different halogen atoms; and
A, B and D represent same or different atom or group selected from hydrogen atom, halogen atoms, R¹, R¹O, R¹S, R¹SO, R¹SO₂, (R¹)₂N, R¹OOC, R¹OR², R¹ON=CH, CN, NO₂, C₂₋₄ lower alkenyl, C₂₋₄ lower alkynyl, C₃₋₇ cycloalkyl, Ph, PhCH₂, PhO, PhCH₂O, PhOR², PhS, PhCH₂S, PhSR², PhCH₂ON=CH, Naph, and Het,
wherein R¹ represents C₁₋₄ lower alkyl which may be optionally substituted by one or more same or different halogen atoms, R² represents C₁₋₄ lower alkylene, Ph represents phenyl which may be optionally substituted by one to five group (s) selected from R¹, R¹O, R¹S, R¹SO, R¹SO₂, (R¹)₂N, R¹OOC, C₃₋₇ cycloalkyl, CN, NO₂, and halogen atoms, or by one Ph, PhCH₂, PhO, PhCH₂O, PhCH₂ON=C(R¹), PhCO, and Het-0; wherein R¹ represents the same as above, Het represents a 5- or 6-membered heteroaromatic group, and two of substituents R¹ and/or R¹O at the vicinal positions may form ring structure with the benzene ring; Naph represents naphthyl which may be optionally substituted by one or two group(s) selected from halogen atoms, R¹ and R¹O, and Het represents the same as above,
provided that A, B and D do not represent hydrogen atom at the same time and more than two of A, B and D do not represent a group containing aromatic or heteroaromatic group at the same time.

2. The compound of claim 1, wherein W represents a C₁₋₃ alkylene group.

3. The compound of claim 1 or 2, wherein W represents a methylene group.

4. The compound of claim 2, wherein X represents R¹OOC or R¹HNOC and Y represents CH, R¹ being defined as in claim 1.

5. A fungicide in agriculture and horticulture and for industrial materials containing one of more kinds of the compounds as claimed in any one of Claims 1 to 4 as active ingredients.

## Patentansprüche

1. Verbindung mit der folgenden allgemeinen Formel (I) : 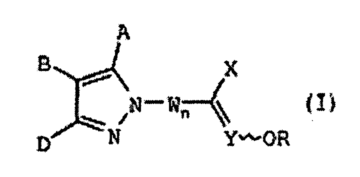 wobei X R¹OOC, R¹HNOC, R¹R¹NOC, CN oder eine 5- oder 6-gliedrige heteroaromatische Gruppe darstellt;
Y CH oder N darstellt;
W C₁₋₃-Alkylen, NR¹ oder 0 darstellt;
n 1 ist;
R eine C₁₋₄-Niederalkylgruppe darstellt, die wahlweise durch ein oder mehrere gleiche oder verschiedene Halogenatome substituiert sein kann; und
A, B und D gleiche oder verschiedene Atome oder Gruppen darstellen, ausgewählt aus einem Wasserstoffatom, Halogenatomen, R¹, R¹O, R¹S, R¹SO, R¹SO₂, (R¹)₂N, R¹OOC, R¹OR², R¹ON=CH, CN, NO₂, C₂₋₄-Niederalkenyl, C₂₋₄-Niederalkinyl, C₃₋₇-Cycloalkyl, Ph, PhCH₂, PhO, PhCH₂O, PhOR², PhS, PhCH₂S, PhSR², PhCH₂ON=CH, Naph und Het,
wobei R¹ C₁₋₄-Niederalkyl darstellt, welches wahlweise durch ein oder mehrere gleiche oder verschiedene Halogenatome substituiert sein kann, R² ein C₁₋₄-Niederalkylen darstellt, Ph ein Phenyl darstellt, welches wahlweise durch ein bis fünf Gruppen substituiert sein kann, ausgewählt aus R¹, R¹O, R¹S, R¹SO, R¹SO₂, (R¹)₂N, R¹OOC, C₃₋₇-Cycloalkyl, CN, NO₂ und Halogenatomen, oder durch ein Ph, PhCH₂, PhO, PhCH₂O, PhCH₂ON=C(R¹), PhCO und Het-0; wobei R¹ das gleiche wie oben bedeutet, Het eine 5-oder 6-gliedrige heteroaromatische Gruppe darstellt, und zwei der Substituenten R¹ und/oder R¹O an den benachbarten Positionen eine Ringstruktur mit dem Benzolring bilden können; Naph eine Naphthylgruppe darstellt, die wahlweise durch ein oder zwei Gruppen substituiert sein kann, ausgewählt aus Halogenatomen, R¹ und R¹O, und Het das gleiche wie oben bedeutet,
mit der Maßgabe, dass A, B und D nicht gleichzeitig ein Wasserstoffatom darstellen, und mehr als zwei von A, B und D nicht gleichzeitig eine Gruppe darstellen, die eine aromatische oder heteroaromatische Gruppe enthält.

2. Verbindung nach Anspruch 1, wobei W eine C₁₋₃₋Alkylengruppe darstellt.

3. Verbindung nach Anspruch 1 oder 2, wobei W eine Methylengruppe darstellt.

4. Verbindung nach Anspruch 2, wobei X R¹OOC oder R¹HNOC darstellt und Y CH darstellt, wobei R¹ wie in Anspruch 1 definiert ist.

5. Fungizid in der Landwirtschaft und Gartenwirtschaft sowie für industrielle Materialien, enthaltend ein oder mehrere Arten der Verbindungen nach einem der Ansprüche 1 bis 4 als aktive Bestandteile.

## Revendications

1. Composé ayant la formule générale suivante (I) : 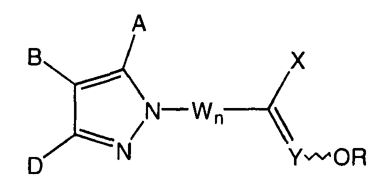 dans laquelle
X représente R¹OOC, R¹HNOC, R¹R¹NOC, CN ou un groupe hétéroaromatique à 5 ou 6 chaînons ;
Y représente CH ou N ;
W représente un groupe alkylène en C₁₋₃, NR¹, ou O ;
n est 1 ;
R représente un groupe alkyle inférieur en C₁₋₄ qui peut éventuellement être substitué par un ou plusieurs atomes d'halogène identiques ou différents ; et
A, B et D représentent des atomes ou groupes identiques ou différents choisis parmi un atome d'hydrogène, des atomes d'halogène, R¹, R¹O, R¹S, R¹SO, R¹SO₂, (R¹)₂N, R¹OOC, R¹OR², R¹ON=CH, CN, NO₂, un groupe alcényle inférieur en C₂₋₄, un groupe alcynyle inférieur en C₂₋₄, un groupe cycloalkyle en C₃₋₇, Ph, PhCH₂, PhO, PhCH₂O, PhOR², PhS, PhCH₂S, PhSR², PhCH₂ON=CH, Naph, et Het,
où R¹ représente un groupe alkyle inférieur en C₁₋₄ qui peut être éventuellement substitué par un ou plusieurs atomes d'halogène identiques ou différents, R² représente un groupe alkylène inférieur en C₁₋₄, Ph représente un groupe phényle qui peut être éventuellement substitué par un à cinq groupe(s) choisi(s) parmi R¹, R¹O, R¹S, R¹SO, R¹SO₂, (R¹)₂N, R¹OOC, un groupe cycloalyle en C₃₋₇, CN, NO₂, et des atomes d'halogène, ou par un Ph, PhCH₂, PhO, PhCH₂O, PhCH₂ON=C(R¹), PhCO, et het-O ; où R¹ représente un groupe identique à celui ci-dessus, Het représente un groupe hétéroaromatique à 5 ou 6 chaînons, et deux des substituants R¹ et/ou R¹O dans les positions vicinales peuvent former une structure cyclique avec le cycle benzène ; Naph représente un groupe naphtyle qui peut être éventuellement substitué par un ou deux groupe(s) choisi(s) parmi des atomes d'halogène, R¹ et R¹O, et Het représente un groupe identique à celui ci-dessus,
à condition que A, B et D ne représentent pas en même temps un atome d'hydrogène et que plus de deux de A, B et D ne représentent pas en même temps un groupe contenant un groupe aromatique ou hétéroaromatique.

2. Composé selon la revendication 1, dans lequel W représente un groupe alkylène en C₁₋₃.

3. Composé selon la revendication 1 ou 2, dans lequel W représente un groupe méthylène.

4. Composé selon la revendication 2, dans lequel X représente R¹OOC ou R¹HNOC et Y représente CH, R¹ étant défini comme dans la revendication 1.

5. Fongicide pour l'agriculture et l'horticulture et pour des matériaux industriels contenant une ou plusieurs sortes des composés tels que revendiqués dans l'une quelconque des revendications 1 à 4 comme ingrédients actifs.
